# EUROPEAN PATENT SPECIFICATION

(11) **EP 1 732 469 B1**
(45) Date of publication and mention of the grant of the patent: **07.07.2010**
(21) Application number: 05729863.0
(22) Date of filing: 04.03.2005
(51) Int. Cl.: A61F 2/06

(54) **BIORESORBABLE STENT WITH BENEFICIAL AGENT RESERVOIRS**
BIORESORBIERBARER STENT MIT RESERVOIRS FÜR VORTEILHAFTE MITTEL
ENDOPROTHESE BIORESORBABLE A RESERVOIRS D'AGENTS BENEFIQUES

(30) Priority: 08.04.2004 US 822063
(43) Date of publication of application: 20.12.2006
(73) Proprietor: Conor Medsystems, Inc., Menlo Park, CA 94025 (US)
(72) Inventor: SHANLEY, John, F., Redwood City, CA 94061 (US); LITVACK, Frank, Los Angeles, CA 90010 (US); PARKER, Theodore, L., Danville, CA 94506 (US); DIAZ, Stephen, Hunter, Palo Alto, CA 94301 (US)
(74) Representative: Schorr, Frank Jürgen
(86) International application number: PCT/US2005/007208
(87) International publication number: WO 2005/102222

(56) References cited:
- WO-A-98/36784
- US-A1- 2002 007 209
- US-A1- 2002 082 680
- US-A1- 2002 107 563
- US-A1- 2003 004 564
- US-B1- 6 241 762
- US-B1- 6 287 332
- US-B1- 6 660 034

## Description

Most coronary artery-related deaths are caused by atherosclerotic lesions which limit or obstruct coronary blood flow to heart tissue. To address coronary artery disease, doctors often resort to percutaneous transluminal coronary angioplasty (PTCA) or coronary artery bypass graft (CABG). PTCA is a procedure in which a small balloon catheter is passed down a narrowed coronary artery and then expanded to re-open the artery. The major advantage of angioplasty is that patients in which the procedure is successful need not undergo the more invasive surgical procedure of coronary artery bypass graft. A major difficulty with PTCA is the problem of post-angioplasty closure of the vessel, both immediately after PTCA (acute reocclusion) and in the long term (restenosis).

Coronary stents are typically used in combination with PTCA to reduce reocclusion of the artery. Stents are introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

While the exact mechanisms of restenosis are still being determined, certain agents have been demonstrated to reduce restenosis in humans. One example of an agent which has been demonstrated to reduce restenosis when delivered from a stent is paclitaxel, a well-known compound that is commonly used in the treatment of cancerous tumors. However, the stents which are currently available and under development for delivery of anti-restenotic agents use surface coatings with suboptimal agent release profiles and side effects. In one example, over 90% of the total agent loaded onto the stent is permanently retained in the stent and is never delivered to the tissue.

There are two types of stents that are presently utilized: permanent stents and bioresorbable stents. A permanent stent is designed to be maintained in a body lumen for an indeterminate amount of time. Permanent stents are typically designed to provide long-term support for damaged or traumatized wall tissues of the lumen. There are numerous conventional applications for permanent stents including cardiovascular, peripheral, urological, gastrointestinal, and gynecological applications.

Bioresorbable stents may advantageously be eliminated from body lumens after a predetermined, clinically appropriate period of time, for example, after the traumatized tissues of the lumen have healed and a stent is no longer needed to maintain the patency of the lumen.

Patent application US 2002/0082680 A1 discloses an expandable medical device having a beneficial agent filled in openings in the device. The benefical agent may be provided in a biodegradable carrier polymer.

Patent application US 2002/0007209 A1 discloses an expandable medical device having a beneficial agent filled in openings in the device. The device may be made from sheet metal or from a biostable or bioabsorbable polymer.

WO 98/36784 A1 discloses a beneficial-agent coated medical device comprising a plurality of recesses. As a material of the device, stainless steel is considered particularly useful, other materials including biodegradable polymers.

Patent document US 6,660,034 B1 discloses an implantable stent coated with a therapeutic substance on its inner and outer surface.

Patent application US 2003/004564 A1 discloses a stent based drug delivery system including a biodegradable polymer matrix entrapping the drug. Independently, the stent may be made from a polymeric biodegradable.

Patent application US 2002/107563 A1 discloses an expandable medical device having a beneficial agent filled in openings in the device. The device may be made from biodegradable materials. The same material may contain the benefical agent.

It is known that the metal stents may become encrusted, encapsulated, endothelialized or ingrown with body tissue. Metal stents could possibly cause irritation to the surrounding tissues in a lumen due to the fact that metals are typically much harder and stiffer than the surrounding tissues in a lumen, which may result in an anatomical or physiological mismatch, thereby damaging tissue or eliciting unwanted biologic responses.

It is known to use bioabsorbable and bioresorbable materials for manufacturing stents. The conventional bioabsorbable or bioresorbable materials from which such stents are made are selected to resorb or degrade over time, thereby eliminating the need for subsequent surgical procedures to remove the stent from the body lumen if problems arise. However, formation of a bioabsorbable stent with a drug within the stent is difficult because the thermoforming processes necessary for formation of the bioabsorbable stent are often not tolerated by the drug. Further, as discussed above, surface coatings on bioabsorbable stents, like the coatings on permanent metal stents have difficulty in controlling the release of the drug due to the limitations of a surface coating.

### Summary of the Invention

The present invention relates to a bioresorbable drug delivery stent comprising a substantially cylindrical expandable stent formed of a bioresorbable material and a plurality of reservoirs formed in the stent containing a beneficial agent matrix comprising a bioresorbable polymer and a drug.

In accordance with one aspect of the present invention, a bioresorbable drug delivery stent includes a substantially cylindrical expandable stent formed of a plurality of struts of a bioresorbable material, a plurality of openings formed in the stent struts, and a beneficial agent matrix loaded within the plurality of openings, the beneficial agent matrix comprising a bioresorbable matrix material drug.

In accordance with another aspect of the present invention, a bioresorbable drug delivery stent includes a substantially cylindrical expandable stent body formed of a bioresorbable material and a plurality of openings formed in the stent body containing a beneficial agent matrix comprising a bioresorbable polymer and a drug, wherein the bioresorbable material of the stent body is a different material than the bioresorbable polymer of the beneficial agent matrix.

In accordance with a further aspect of the invention, a method of reducing restenosis with a bioresorbable drug delivery stent, includes the steps of providing a drug delivery bioresorbable stent having a dosage of anti-restenotic drug arranged within a plurality of openings in the stent without coating an exterior surface of the stent with the anti-restenotic drug, implanting the stent within an artery of a patient, and delivering the anti-restenotic drug from the stent to the artery at a minimum release rate of 1 percent of the total dosage of the drug on the stent per day throughout an entire administration period from the time of implantation of the stent until the time that substantially all the drug is released from the stent.

In accordance with an additional aspect of the invention, a bioresorbable drug delivery stent includes a substantially cylindrical expandable stent formed of a bioresorbable material, a plurality of openings formed in the stent, and a beneficial agent matrix loaded within the plurality of openings, the beneficial agent matrix comprising a drug. The beneficial agent matrix is arranged such that the beneficial agent matrix does not block access of fluid from an environment surrounding the stent to the bioresorbable stent material.

### Brief Description of the Drawings

The invention will now be described in greater detail with reference to the preferred embodiments illustrated in the accompanying drawings, in which like elements bear like reference numerals, and wherein:
FIG. 1 is a perspective view of one example of a stent according to the present invention.
FIG. 2 is a side view of a portion of the stent of FIG. 1.
FIG. 3 is a side view of a portion of another example of a stent woven from filaments.
FIG. 4 is a side view of a portion of another example of a stent with a lattice configuration.
FIG. 5 is a side cross sectional view of an example of an opening in a stent showing a matrix with a therapeutic agent and a barrier layer.
FIG. 6 is a side cross sectional view of another example of an opening in a stent showing a matrix with two therapeutic agents.

### Detailed Description

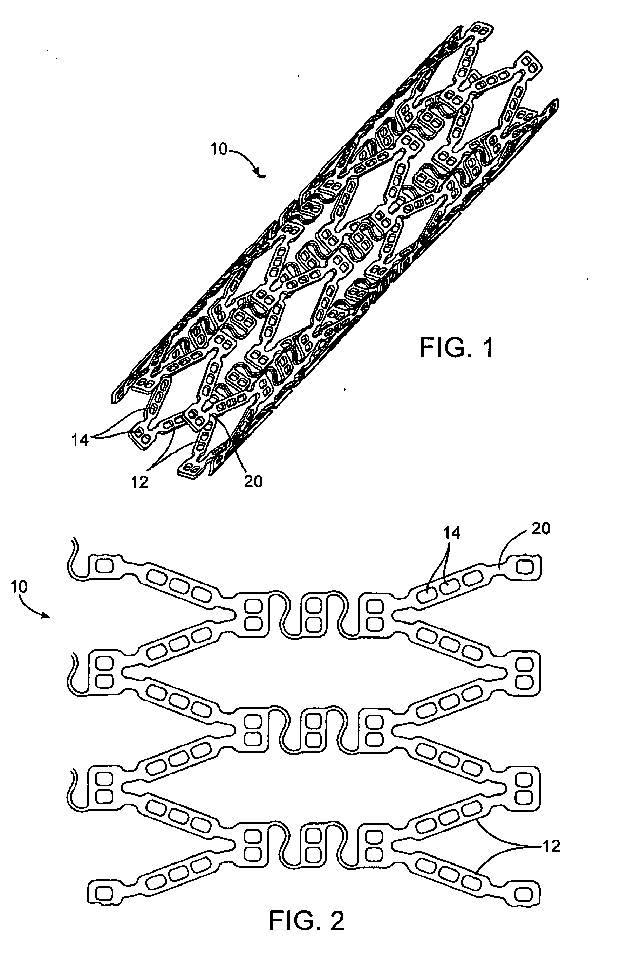

A biodegradable or bioresorbable drug delivery stent as illustrated in FIGS. 1-4 of the present invention includes a substantially cylindrical expandable stent formed of a bioresorbable material and a plurality of reservoirs formed in the stent containing a beneficial agent matrix. The bioresorbable stent material can be a bioresorbable metal alloy, a bioresorbable polymer, a bioresorbable composite or the like which has sufficient structural integrity to support a lumen, such as a blood vessel lumen for a predetermined period of time. The reservoirs containing the beneficial agent matrix allow delivery of the beneficial agent, such as an antirestenotic drug, for an administration period which is generally equal to or less than a time that the bioresorbable stent is retained in the lumen. The beneficial agent matrix may include one or more bioresorbable polymers or other matrix materials in combination with one or more therapeutic agents or drugs.

The following terms, as used herein, shall have the following meanings:

The terms "drug" and "therapeutic agent" are used interchangeably to refer to any therapeutically active substance that is delivered to a living being to produce a desired, usually beneficial, effect.

The term "beneficial agent" as used herein is intended to have its broadest possible interpretation and is used to include any therapeutic agent or drug, as well as inactive agents such as barrier layers, carrier layers, therapeutic layers, or protective layers.

The term "matrix" or "biocompatible matrix" are used interchangeably to refer to a medium or material that, upon implantation in a subject, does not elicit a detrimental response sufficient to result in the rejection of the matrix. The matrix may contain or surround a therapeutic agent, and/or modulate the release of the therapeutic agent into the body. A matrix is also a medium that may simply provide support, structural integrity or structural barriers. The matrix may be polymeric, non-polymeric, hydrophobic, hydrophilic, lipophilic, amphiphilic, crystalline and the like.

The term "bioresorbable" refers to a material, as defined herein, that can be broken down by either chemical or physical process, upon interaction with a physiological environment. The bioresorbable material can erode or dissolve. A bioresorbable material serves a temporary function in the body, such as supporting a lumen or drug delivery, and is then degraded or broken into components that are metabolizable or excretable, over a period of time from minutes to years, preferably less than one year, while maintaining any requisite structural integrity in that same time period.

The term "openings" includes both through openings and recesses.

The term "pharmaceutically acceptable" refers to the characteristic of being non-toxic to a host or patient and suitable for maintaining the stability of a therapeutic agent and allowing the delivery of the therapeutic agent to target cells or tissue.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{W} greater than about 3000 and preferably greater than about 10,000 and a M_{W} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.

The term "primarily" with respect to directional delivery, refers to an amount greater than about 50% of the total amount of therapeutic agent provided to a blood vessel.

The term "restenosis" refers to the renarrowing of an artery following an angioplasty procedure which may include stenosis following stent implantation.

The term "substantially linear release profile" refers to a release profile defined by a plot of the cumulative drug released versus the time during which the release takes place in which the linear least squares fit of such a release profile plot has a correlation coefficient value, r², of greater than 0.92 for data time points after the first day of delivery.

FIG. 1 illustrates one example of an implantable medical device in the form of a biodegradable or bioresorbable stent 10. FIG. 2 is an enlarged flattened view of a portion of the stent of FIG. 1 illustrating one example of a stent structure including struts 12 interconnected by ductile hinges 20. The struts 12 include openings 14 which can be non-deforming openings containing a therapeutic agent. One example of a stent structure having non-deforming openings is shown in U.S. Patent No. 6,562,065 which is incorporated herein by reference in its entirety.

The bioresorbable stent 10 can be formed of a bioresorbable metal alloy, a bioresorbable polymer. Bioresorbable metal alloys useful for stents include zinc-titanium alloys, and magnesium alloys, such as lithium-magnesium, sodium-magnesium, and magnesium alloys containing rare earth metals. Some examples of bioresorbable metal alloys are described in U.S. Patent No. 6,287,332. Bioresorbable metal alloy stents can be formed in the configuration illustrated in FIGS. 1 and 2 by laser cutting. When cutting stents from these alloys, an inert atmosphere may be desired to minimize oxidation of the alloy during cutting in which case, a helium gas stream, or other inert atmosphere can be applied during cutting. Magnesium alloys are used in the aeronautic industry and the processing systems used for the aeronautic industry can also be used for forming the stents. Bioresorbable metal alloys provide the necessary structural strength needed for the stent, however, it is difficult to incorporate a drug within the bioresorbable metal alloy and is difficult to release the drug if it could be incorporated.

More importantly, the use of coatings on the bioresorbable metal alloy surface containing a drug may interfere with the biodegradation of the stent. Therefore, the present invention of providing openings in the bioresorbable stent and filling the openings with a bioresorbable matrix containing drug provides a solution because there is no requirement for a coating on the stent.

When the bioresorbable stent 10 is formed of a bioresorbable polymer material, similar problems can occur when attempting to adding a drug to the stent by incorporating drug into the polymer or coating drug onto the stent. For example, bioresorbable polymers which have sufficient strength to be used as a stent may not be capable of incorporating a drug and releasing the drug in a desired manner. Further, drug coatings require that they adhere well without cracking or flaking during delivery and also release the drug in a desired manner. Additionally, polymer stents tend to have high recoil.

Another difficulty in incorporating drugs in polymer stents is that methods for forming bioresorbable polymer stents tend to be high temperature processes which are not suitable for many drugs. With polymer stents, as with bioresorbable metal alloys, a coating may also interfere with bioresorbtion of the stent.

The bioresorbable stent of the present application provides a solution to these problems by selecting a first bioresorbable polymer for the struts of the stent and providing openings in the stent containing a beneficial agent matrix. The polymer or other matrix material in the openings require none of the structural properties of the stent, and also require very little flexibility or adhesion which is required by a coating. Thus, the matrix material selection may be made based on the ability of the material to release the drug with a desired release profile. Directional delivery of one or more drugs can also be achieved with reservoirs which cannot be easily achieved with coatings, impregnation, or other methods.

Examples of bioresorbable polymers which can be used for the structural struts of the stent 10 include, without limitation, polylactic acid (PLA), polyglycolic acid (PGA), copolymers of PLA and PGA, poly-L-lactide (PLLA), poly-D,L-lactide (PDLA), poly-ε-capralactone (PCL), and combinations thereof. U.S. Patent No. 4,889,119 describes some of the bioresorbable polymers which are useful in the present invention.

Examples of bioresorbable polymers which can be used tor the polymer/drug matrix within the reservoirs include, without limitation, polylactic acid (PLA); polyglycolic acid (PGA); copolymers of PLA and PGA; polylactic-co-glycolic acid (PLGA); poly-L-lactide (PLLA); poly-D,L-lactide (PDLA); poly-∈ capralactone (PCL); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; and combinations thereof. Preferably, the polymer in the reservoir degrades at a rate which results in degradation of the matrix substantially at the same time or before the degradation of the stent itself.

Bioresorbable polymer stents can be formed by known methods including molding, extrusion, other thermoforming processes, laser cutting, semiconductor fabrication methods including microdischarge machining or a combination of these processes. Laser cutting of a polymer tube to form a stent 10, such as the stent illustrated in FIGS. 1 and 2, can be performed with a UV laser, excimer laser or other known laser. The stent illustrated in FIGS. 1 and 2 is only one example of the type of stent structure which may be made. Many other stent configurations can also be used including woven stents, coil stents, serpentine patterns, diamond patterns, chevron or other patterns, or racheting or locking stents.

Molds for forming bioresorbable polymer stents can be formed by a number of know methods including photolithography, EMD, other semiconductor fabrication processes, degradable molds, lost wax casting, or the like. For example, in one process, a stent form can be created by photolithography, a silicon rubber mold can be formed from the stent form, and the rubber mold can be metalized to created the rigid stent mold useful for molding the polymer stents under high pressure. The stent 10 can be molded with the openings 14 formed during the molding step. Alternatively, the openings 14 can be formed in a later step, such as by laser cutting.

The mold used to form the stent may include a central pin or core and two or more surrounding removable mold members. The molded stents can be removed from the core by one of several methods including mechanically by lifting pins or wires, pneumatically by passage of air under the stents, or by swelling the plastic by application of a liquid, such as a solvent to a swellable material, such as a cross-linked polymer. Alternatively, the core can be formed of a collapsible configuration.

Although the openings 14 have been illustrated as through holes, other shaped openings including recesses, channels, wells, and grooves can be easily formed by a molding process.

Although similar bioresorbable polymers can be used for the stent structure and the polymer/drug matrix in the openings, these polymers are formed in different ways. The stent polymer is formed by a high temperature forming process, for example, temperatures of above 100 degrees C and preferably above 120 degrees C can be required for forming the stent. However, since these high temperatures cause degradation of most drugs, the polymer of the polymer/drug matrix is formed by a different process, such as with the use of a solvent at a lower temperature which is generally below 100 degrees C, and preferably below about 75 degrees C. The present invention separates the step of forming the structural portion of the stent from the step of forming the drug delivery portion of the stent without requiring a coating.

The bioresorbable material of the matrix and any other materials within the reservoirs can be delivered into the openings in a liquidified state which can be achieved by either a solvent or an elevated temperature. When a solvent is used to deliver the matrix solution into the openings, the solvent selected should be a solvent which does not substantially degrade the bioresorbable material of the stent. For example, a stent formed of PLLA can be formed with openings which can be filled with a solution comprising PLGA, DMSO, and drug. The DMSO will not appreciably degrade the PLLA of the stent and will be evaporated to form the polymer/drug matrix within the openings. In another example, the polymer of the stent can be cross-linked, coated, or otherwise treated to prevent the solvent from degrading the polymer.

In a further example, a stent formed of PLGA can include openings which are filled with a hydrophilic polymer (PEO, PVP, dextrin) and a hydrophilic drug (insulin) dissolved in water.

The bioresorbable polymer and bioresorbable metal alloy stents can be either balloon expandable or self expanding. For example, self expanding polymer stents may be formed in an expanded configuration and compressed for delivery within a delivery system which constrains the stent. When the delivery system constrains are removed, the stent returns to the expanded size. In another example, a self expanding polymer stent can be retained on a balloon catheter by a breakable or erodible constraining mechanism, such as a thread. Upon delivery of the balloon catheter to a desired implantation position within a lumen, the balloon is expanded, thus breaking the thread and allowing the stent to expand to support the lumen.

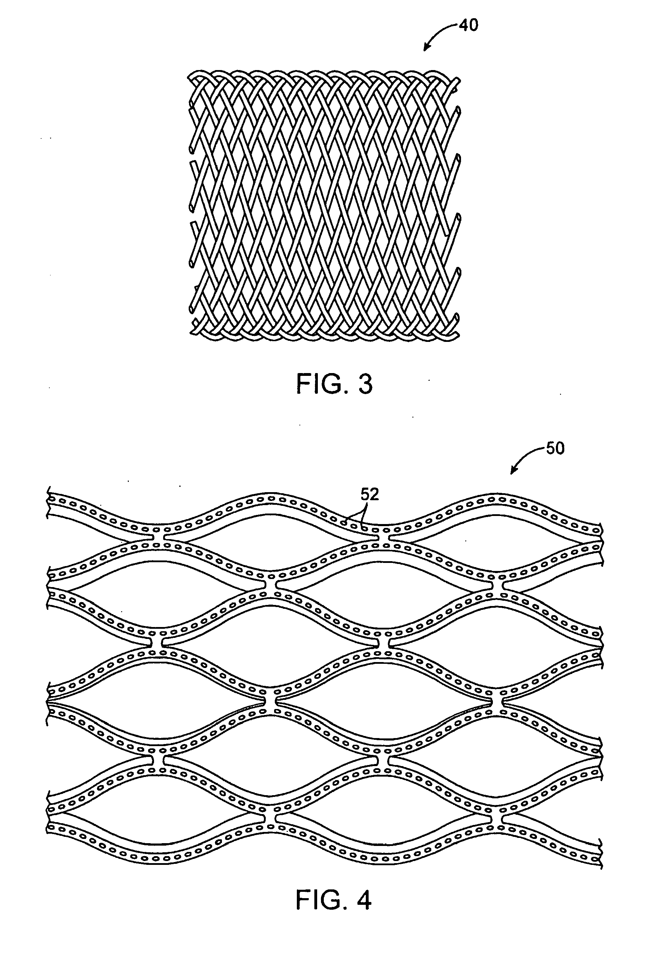

FIG. 3 illustrates an alternative embodiment of a bioresorbable stent 40 which is woven from a bioresorbable wire. The bioresorbable wire may be any of the bioresorbable metal alloys, bioresorbable polymer materials, or other bioresorbable materials described above. In the mesh stent, reservoirs are formed in the wires of the mesh either before or after weaving the wires into the mesh. The reservoirs can also be filled with the polymer/drug matrix either before or after weaving.

In a second embodiment, the bioresorbable wire mesh stent 40 of FIG. 3 can be woven and then compressed under application of heat to form the mesh into a single layer of lattice with gaps or diamond shaped openings between the lattice members. These gaps or openings are then filled with the bioresorbable drug delivery matrix to form the drug delivery stent.

FIG. 4 illustrates another embodiment of a bioresorbable stent 50 which can be extruded, molded, or laser cut in a lattice structure. The openings 52 can be formed in the lattice structure of the stent 50 either during the process of forming the stent or subsequently. The openings 52 are then filled with the polymer/drug matrix.

### The Beneficial Agent Matrix Formation

The bioresorbable stents of the present invention are configured to release at least one therapeutic agent from the matrix contained in reservoirs in the implantable stent body. The matrix is formed such that the distribution of the agent in the polymer matrix as well as barrier layers, protective layers, separating layers, and cap layers which form a part of the matrix together control the rate of elution of the agent from the reservoirs.

In one embodiment, the matrix is a polymeric material which acts as a binder or carrier to hold the agent in the stent and/or modulate the release of the agent from the stent. The drug will be held within the reservoirs in the stent in a drug delivery matrix comprised of the drug and a polymeric or other material and optionally additives to regulate the drug release.

The therapeutic agent containing matrix can be disposed in the stent in various configurations, including within volumes defined by the stent, such as openings, holes, grooves, channels, or concave surfaces, as a reservoir of agent. When the therapeutic agent matrix is disposed within openings in the strut structure of the stent to form a reservoir, the openings may be partially or completely filled with matrix containing the therapeutic agent. The beneficial agent matrix when fixed to the stent is arranged such that it does riot block access of fluid from the surrounding environment to the bioresorbable stent or otherwise appreciable change the bioresorbtion of the stent.

The beneficial agent matrix within the openings may be formed by one of a plurality of methods. One such method is described in US 2004/0127976 A1. According to this method the matrix is loaded into the openings by forming a solution of polymer, drug, and solvent, and delivering the solution into the openings by a piezoelectric dispenser in a plurality of steps which form multiple individual or intermixing layers with different chemical and/or pharmacological properties.

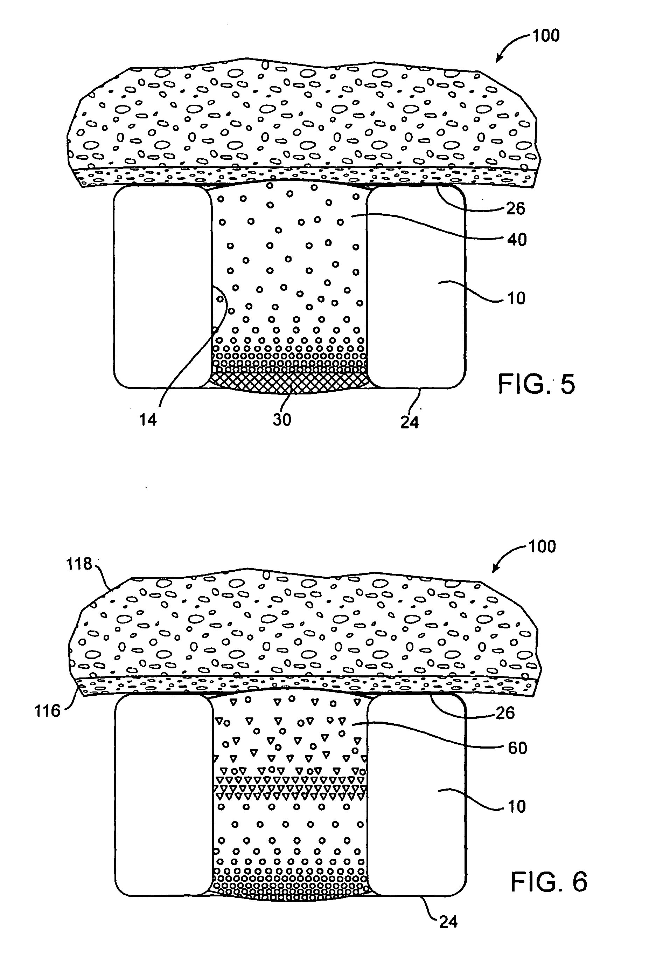

FIG. 5 is a cross section of one strut of the stent 10 and a blood vessel 100 illustrating one example of a through opening 14 arranged adjacent the vessel wall with a mural surface 26 abutting the vessel wall and a luminal surface 24 opposite the mural surface. The opening 14 of FIG. 3 contains a matrix 40 with a therapeutic agent illustrated by Os in the matrix. The luminal side 24 of the stent opening 14 is provided with a barrier layer 30. The barrier layer 30 erodes more slowly than the matrix 40 containing the therapeutic agent and thus, causes the therapeutic agent to be delivered primarily to the mural side 26 of the stent. The matrix 40 and therapeutic agent are arranged in a programmable manner to achieve a desire release rate and administration period. As can be seen in the example of FIG. 5, the concentration of the therapeutic agent (Os) is highest adjacent the barrier layer 30 of the stent 10 and lowest at the mural side 26 of the stent. This configuration in which the drug can be precisely arranged within the matrix allows the release rate and administration period to be selected and programmed to a particular application. The methods by which the drug can be precisely arranged within the matrix in the openings is a stepwise deposition process and is further described in US 2005/0010170 A1.

FIG. 6 is a cross section of a strut of the stent 10 having an opening 14 in which a polymer/drug matrix 60 includes a first drug illustrated by Os and second drug illustrated by 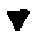 s. The two drugs may be located in separate regions of the matrix or intermixed (as shown) to achieve different release profiles and administration periods for the two drugs.

Numerous other useful arrangements of the matrix and therapeutic agent can be formed to achieve different release rates including substantially linear release, substantially first order release, pulsitile release, or any other desired release. The arrangement of the polymer and agent in the matrix also controls the duration of release or administration period which may be a short release of 1-24 hours, moderate release of about 1 to about 7 days, or extended release of about 7 or more days, preferably about 30 days. Each of the areas of the matrix may include one or more agents in the same or different proportions from one area to the next. The matrix may be solid, porous, or filled with other drugs or excipients. The agents may be homogeneously disposed or heterogeneously disposed in different areas of the matrix.

When an anti-restenotic agent delivered by the method of the invention is paclitaxel, the total amount delivered (and loaded) is preferably between 2 micrograms and 50 micrograms. In one preferred embodiment, the amount of paclitaxel delivered will be between about 0.1 micrograms and about 15 micrograms on the first day, more preferably between about 0.3 micrograms and about 9 micrograms. Following day one, the paclitaxel will be delivered in a substantially linear fashion at a rate of about 0.025 micrograms to about 2.5 microgram per day for a minimum of 21 days, preferably about 0.2 to about 2 micrograms per day. It is envisioned that all the paclitaxel will be released from the stent in less than 60 days. The total amount of paclitaxel loaded onto the stent and released into the tissue in need of treatment is envisioned to be preferably in the range of about 1.5 micrograms to about 75 micrograms, preferably about 3 to about 30 micrograms. The above release rates for paclitaxel have been given for a standard stent of dimensions 3.0 mm in expanded diameter by 17 mm in length. Stents of other dimensions are envisioned to contain total drug loadings in similar respective proportions based on similar drug loading density or drug per unit length. In one example, the amount of paclitaxel released per day after day one is about 0.0003 to about 0.03 ug/mm² of tissue surface area, preferably about 0.0003 to about 0.01 ug/mm² of tissue surface area. In another example, the amount of paclitaxel released per day after day one is about 0.001 to about 0.2 ug/mm of stent length per day.

The methods of the invention preferably will result in sustained release of substantially all the drug loaded onto the stent in no longer than 180 days, preferably in no longer than 60 days, and most preferably in no longer than 35 days.

It is envisioned that all beneficial agent matrix will be bioresorbed in about 14 days to about one year, more preferably in about 30 days to about 90 days. It is also envisioned that stent structure will be bioresorbed in about 20 days to about 365 days, preferably about 30 days to about 180 days.

### Therapeutic Agents

The present invention relates to the delivery of anti-restenotic agents including paclitaxel, rapamycin, cladribine, and their derivatives, as well as other cytotoxic or cytostatic agents and microtubule stabilizing agents. The present invention may also be used to deliver other agents alone or in combination with anti-restenotic agents. Some of the other agents delivered either alone or in combination may be those that to reduce tissue damage after myocardial infarction, stabilize vulnerable plaque, promote angiogenesis, or reduce inflammatory response.

Other therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, AZ blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, paclitaxel, actinomycin D, rapamycin, and cyclosporin.

Antithrombins include, without limitation, heparin, plasminogen, α₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation; glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limitation, Adiponectin.

While the invention has been described in detail with reference to the preferred embodiments thereof, it will be apparent to one skilled in the art that various changes and modifications can be made and equivalents employed, without departing from the present invention.

## Claims

1. A bioresorbable drug delivery stent comprising a substantially cylindrical expandable stent body and a plurality of openings formed in the stent body containing a beneficial agent matrix comprising a matrix material and a drug, wherein the material of the stent body is a different material than the material of the beneficial agent matrix,
the stent body is formed of a bioresorbable material and the material comprised in the matrix is bioresorbable, **characterized in that** the bioresorbable material of the stent comprises a material which degrades more slowly than the bioresorbable matrix material of the beneficial agent matrix.

2. The stent of Claim 1, wherein the bioresorbable material of the stent comprises a polymer having a strength greater than the bioresorbable matrix material of the beneficial agent matrix.

3. The stent of Claim 1, wherein the bioresorbable material of the stent comprises a material which is not significantly soluble by a solvent in which the bioresorbable matrix material of the beneficial agent matrix is soluble.

4. The bioresorbable drug delivery stent according to claim 1, wherein the beneficial agent matrix is arranged such that the beneficial agent matrix does not block access of fluid from an environment surrounding the stent to the bioresorbable stent material.

5. The stent of Claim 4, wherein the bioresorbable material of the stent comprises a polymer having a strength greater than the beneficial agent matrix.

6. The stent of Claim 4, wherein the bioresorbable material of the stent comprises a material which is not significantly soluble by a solvent in which the beneficial agent matrix is soluble.

7. The stent of Claim 1 or 4, wherein the bioresorbable material of the stent is a bioresorbable metal alloy.

8. The stent of Claim 1 or 4, wherein the bioresorbable material of the stent is a bioresorbable polymer.

9. The stent of Claim 1 or 4, wherein the beneficial agent matrix comprises a bioresorbable polymer.

10. The stent of Claim 1 or 4, wherein an anti-restenotic drug is contained in the openings in the bioresorbable stent.

11. The stent of Claim 10, wherein the anti-restenotic drug is contained in the openings in the bioresorbable polymer matrix.

12. The stent of Claim 10, wherein the anti-restenotic drug and bioresorbable polymer matrix are contained in the openings forming a matrix within the openings which has a concentration gradient.

13. A method of manufacturing a bioresorbable drug delivery stent, comprising forming a substantially cylindrical expandable stent, forming a plurality of openings in the stent, and providing a beneficial agent matrix comprising a matrix material and a drug in the openings, wherein the material of the stent is a different material than the material of the beneficial agent matrix,
**characterized by**
forming the stent body of a bioresorbable material which degrades more slowly than a bioresorbable matrix material of the beneficial agent matrix.

14. The method of Claim 13, wherein the bioresorbable material of the stent is formed at a temperature above 100 degrees C and the bioresorbable matrix material of the beneficial agent matrix is formed at a temperature below 100 degrees C.

15. The method of Claim 13, wherein the stent is formed by laser cutting.

16. The method of Claim 13, wherein the stent is formed by molding.

17. The method of Claim 13, wherein the stent is formed by thermoforming.

18. The method of Claim 13, wherein the openings are formed by laser cutting.

19. The method of Claim 13, wherein the openings are formed by molding.

20. The method of Claim 13, wherein the openings are formed by thermoforming.

21. The method of one of claims 13 to 20, comprising arranging the beneficial agent matrix such that the beneficial agent matrix does not block access of fluid from an environment surrounding the stent to the bioresorbable stent material.

22. The method of Claim 13, comprising delivering the bioresorbable material of the matrix and any other material within the openings thereinto in a liquefied state.

23. The method of Claim 22, wherein the liquefied state is achieved by a solvent or else an elevated temperature.

24. The method of Claim 23, wherein the matrix is loaded into the openings by forming a solution of polymer, drug, and solvent, and delivering the solution into the openings by a piezoelectric dispenser in a plurality of steps which form multiple individual or intermixing layers with different chemical and/or pharmacological properties.

## Patentansprüche

1. Bioresorbierbarer Arzneimittelabgabe-Stent, umfassend einen im Wesentlichen zylindrischen expandierbaren Stent-Körper und eine Mehrzahl von Öffnungen, die in dem Stent-Körper gebildet sind, enthaltend eine Matrix eines nützlichen Agens, umfassend ein Matrixmaterial und ein Arzneimittel, wobei das Material des Stent-Körpers ein anderes Material ist als das Material der Matrix des nützlichen Agens,
wobei der Stent-Körper aus einem bioresorbierbaren Material gebildet ist und das Material, das in der Matrix enthalten ist, bioresorbierbar ist,
**dadurch gekennzeichnet, dass** das bioresorbierbare Material des Stents ein Material umfasst, das sich langsamer abbaut als das bioresorbierbare Matrixmaterial der Matrix des nützlichen Agens.

2. Stent gemäß Anspruch 1, wobei das bioresorbierbare Material des Stents ein Polymer mit einer größeren Festigkeit als das bioresorbierbare Matrixmaterial der Matrix des nützlichen Agens umfasst.

3. Stent gemäß Anspruch 1, wobei das bioresorbierbare Material des Stents ein Material umfasst, das durch ein Lösemittel, in dem das bioresorbierbare Matrixmaterial der Matrix des nützlichen Agens löslich ist, nicht signifikant löslich ist.

4. Bioresorbierbarer Arzneimittelabgabe-Stent gemäß Anspruch 1, wobei die Matrix des nützlichen Agens so angeordnet ist, dass die Matrix des nützlichen Agens den Zugang von Fluid von einer Umgebung, die den Stent umgibt, zu dem bioresorbierbaren Stent-Material nicht blockiert.

5. Stent gemäß Anspruch 4, wobei das bioresorbierbare Material des Stents ein Polymer mit einer größeren Festigkeit als die Matrix des nützlichen Agens umfasst.

6. Stent gemäß Anspruch 4, wobei das bioresorbierbare Material des Stents ein Material umfasst, das durch ein Lösemittel, in dem die Matrix des nützlichen Agens löslich ist, nicht signifikant löslich ist.

7. Stent gemäß Anspruch 1 oder 4, wobei das bioresorbierbare Material des Stents eine bioresorbierbare Metalllegierung ist.

8. Stent gemäß Anspruch 1 oder 4, wobei das bioresorbierbare Material des Stents ein bioresorbierbares Polymer ist.

9. Stent gemäß Anspruch 1 oder 4, wobei die Matrix des nützlichen Agens ein bioresorbierbares Polymer umfasst.

10. Stent gemäß Anspruch 1 oder 4, wobei ein antirestenotisches Arzneimittel in den Öffnungen in dem bioresorbierbaren Stent enthalten ist.

11. Stent gemäß Anspruch 10, wobei das antirestenotische Arzneimittel in den Öffnungen in der bioresorbierbaren Polymermatrix enthalten ist.

12. Stent gemäß Anspruch 10, wobei das antirestenotische Arzneimittel und die bioresorbierbare Polymermatrix in den Öffnungen enthalten sind, wobei sie eine Matrix innerhalb der Öffnungen bilden, die einen Konzentrationsgradienten hat.

13. Verfahren zur Herstellung eines bioresorbierbaren Arzneimittelabgabe-Stents, umfassend das Bilden eines im Wesentlichen zylindrischen expandierbaren Stents, das Bilden einer Mehrzahl von Öffnungen in dem Stent und das Bereitstellen einer Matrix des nützlichen Agens, umfassend ein Matrixmaterial und ein Arzneimittel in den Öffnungen, wobei das Material des Stents ein anderes Material ist als das Material der Matrix des nützlichen Agens,
**gekennzeichnet durch**
das Bilden des Stent-Körpers aus einem bioresorbierbaren Material, das sich langsamer abbaut als ein bioresorbierbares Matrixmaterial der Matrix des nützlichen Agens.

14. Verfahren gemäß Anspruch 13, wobei das bioresorbierbare Material des Stents bei einer Temperatur über 100 Grad C gebildet wird und das bioresorbierbare Matrixmaterial der Matrix des nützlichen Agens bei einer Temperatur unter 100 Grad C gebildet wird.

15. Verfahren gemäß Anspruch 13, wobei der Stent durch Laser-Schneiden gebildet wird.

16. Verfahren gemäß Anspruch 13, wobei der Stent durch Formen gebildet wird.

17. Verfahren gemäß Anspruch 13, wobei der Stent durch Thermoformen gebildet wird.

18. Verfahren gemäß Anspruch 13, wobei die Öffnungen durch Laser-Schneiden gebildet werden.

19. Verfahren gemäß Anspruch 13, wobei die Öffnungen durch Formen gebildet werden.

20. Verfahren gemäß Anspruch 13, wobei die Öffnungen durch Thermoformen gebildet werden.

21. Verfahren gemäß einem der Ansprüche 13 bis 20, umfassend die Anordnung der Matrix des nützlichen Agens derart, dass die Matrix des nützlichen Agens den Zugang von Fluid von einer Umgebung, die den Stent umgibt, zu dem bioresorbierbaren Stent-Material nicht blockiert.

22. Verfahren gemäß Anspruch 13, umfassend die Gabe des bioresorbierbaren Materials der Matrix und jedes anderen Materials innerhalb der Öffnungen darin in einem verflüssigten Zustand.

23. Verfahren gemäß Anspruch 22, wobei der verflüssigte Zustand durch ein Lösemittel oder sonst eine erhöhte Temperatur erreicht wird.

24. Verfahren gemäß Anspruch 23, wobei die Matrix in die Öffnungen geladen wird, indem eine Lösung aus Polymer, Arzneimittel und Lösemittel gebildet wird, und die Lösung in die Öffnungen durch einen piezoelektrischen Spender in mehreren Schritten abgegeben wird, die mehrere einzelne oder untereinander vermischte Schichten mit verschiedenen chemischen und/oder pharmakologischen Eigenschaften bilden.

## Revendications

1. Stent biorésorbable pour la libération d'un médicament, comprenant un corps de stent dilatable, sensiblement cylindrique, et une pluralité d'ouvertures formées dans le corps de stent contenant une matrice d'agent bénéfique comprenant un matériau de matrice et un médicament, le matériau du corps de stent étant un matériau différent du matériau de la matrice d'agent bénéfique, le corps de stent étant formé d'un matériau biorésorbable et le matériau compris dans la matrice étant biorésorbable, **caractérisé en ce que** le matériau biorésorbable du stent comprend un matériau qui se dégrade plus lentement que le matériau de la matrice biorésorbable de la matrice d'agent bénéfique.

2. Stent selon la revendication 1, dans lequel le matériau biorésorbable du stent comprend un polymère ayant une résistance supérieure à celle du matériau de la matrice biorésorbable de la matrice d'agent bénéfique.

3. Stent selon la revendication 1, dans lequel le matériau biorésorbable du stent comprend un matériau qui n'est pas significativement solubilisé par un solvant dans lequel le matériau de la matrice biorésorbable de la matrice d'agent bénéfique est soluble.

4. Stent biorésorbable pour la libération d'un médicament, selon la revendication 1, dans lequel la matrice d'agent bénéfique est agencée de telle façon que la matrice d'agent bénéfique ne bloque pas l'accès d'un fluide depuis un environnement entourant le stent vers le matériau du stent biorésorbable.

5. Stent selon la revendication 4, dans lequel le matériau biorésorbable du stent comprend un polymère ayant une résistance supérieure à celle de la matrice d'agent bénéfique.

6. Stent selon la revendication 4, dans lequel le matériau biorésorbable du stent comprend un matériau qui n'est pas significativement solubilisé par un solvant dans lequel la matrice d'agent bénéfique est soluble.

7. Stent selon la revendication 1 ou 4, dans lequel le matériau biorésorbable du stent est un alliage métallique biorésorbable.

8. Stent selon la revendication 1 ou 4, dans lequel le matériau biorésorbable du stent est un polymère biorésorbable.

9. Stent selon la revendication 1 ou 4, dans lequel la matrice d'agent bénéfique comprend un polymère biorésorbable.

10. Stent selon la revendication 1 ou 4, dans lequel un médicament anti-resténose est contenu dans les ouvertures du stent biorésorbable.

11. Stent selon la revendication 10, dans lequel le médicament anti-resténose est contenu dans les ouvertures de la matrice de polymère biorésorbable.

12. Stent selon la revendication 10, dans lequel le médicament anti-resténose et le polymère biorésorbable sont contenus dans les ouvertures formant, au sein des ouvertures, une matrice qui a un gradient de concentration.

13. Procédé de fabrication d'un stent biorésorbable pour le libération d'un médicament, comprenant la formation d'un stent dilatable, sensiblement cylindrique, la formation d'une pluralité d'ouvertures dans le stent, et la fourniture d'une matrice d'agent bénéfique comprenant un matériau de matrice et un médicament dans les ouvertures, le matériau du stent étant un matériau différent du matériau de la matrice d'agent bénéfique,
**caractérisé par**
la formation d'un corps de stent en un matériau biorésorbable qui se dégrade plus lentement que le matériau de la matrice biorésorbable de la matrice d'agent bénéfique.

14. Procédé selon la revendication 13, dans lequel le matériau biorésorbable du stent est formé à une température supérieure à 100°C et le matériau de la matrice biorésorbable de la matrice d'agent bénéfique est formé à une température inférieure à 100°C.

15. Procédé selon la revendication 13, dans lequel le stent est formé par découpe au laser.

16. Procédé selon la revendication 13, dans lequel le stent est formé par moulage.

17. Procédé selon la revendication 13, dans lequel le stent est formé par thermoformage.

18. Procédé selon la revendication 13, dans lequel les ouvertures sont formées par découpe au laser.

19. Procédé selon la revendication 13, dans lequel les ouvertures sont formées par moulage.

20. Procédé selon la revendication 13, dans lequel les ouvertures sont formées par thermoformage.

21. Procédé selon l'une quelconque des revendications 13 à 20, comprenant l'agencement de la matrice d'agent bénéfique de telle sorte que la matrice d'agent bénéfique ne bloque pas l'accès d'un fluide depuis un environnement entourant le stent vers le matériau du stent biorésorbable.

22. Procédé selon la revendication 13, comprenant la distribution du matériau biorésorbable de la matrice et tout autre matériau au sein des ouvertures à l'état liquéfié.

23. Procédé selon la revendication 22, dans lequel l'état liquéfié est obtenu au moyen d'un solvant, ou autre, à une température élevée.

24. Procédé selon la revendication 23, dans lequel la matrice est chargée dans les ouvertures en formant une solution de polymère, de médicament et de solvant, et la distribution de la solution dans les ouvertures par un distributeur piézoélectrique en une pluralité d'étapes qui forment de multiples couches individuelles ou entremêlées ayant différentes propriétés chimiques et/ou pharmacologiques.
